(19) Europäisches Patentamt
European Patent Office
Office européen des brevets

(11) Publication number: **0 431 171 B1**

(12) # EUROPEAN PATENT SPECIFICATION


(45) Date of publication of patent specification: **21.06.95**

(51) Int. Cl.6: **C12P 21/08**, C12N 5/20, G01N 33/577, //C12N15/06, (C12P21/08,C12R1:91)

(21) Application number: **90906370.3**

(22) Date of filing: **25.04.90**

(86) International application number: **PCT/JP90/00538**

(87) International publication number: **WO 90/12884 (01.11.90 90/25)**


(54) **MONOCLONAL ANTIBODY AGAINST C-REACTIVE PROTEIN.**


(30) Priority: **25.04.89 JP 103471/89**

(43) Date of publication of application: **12.06.91 Bulletin 91/24**

(45) Publication of the grant of the patent: **21.06.95 Bulletin 95/25**

(84) Designated Contracting States: **DE ES FR GB IT**


(56) References cited:
**WO-A-89/08261**
**JP-A- 6 236 399**
**JP-A-62 210 984**
**JP-A-62 210 985**

**HYBRIDOMA, vol. 7, no. 2, 1988); J. TSENG et al., pp. 185-191/**

**JOURNAL OF IMMUNOLOGY, vol. 131, no. 5, 1983; K. H. ROUX et al., pp. 2411-2415/**


(73) Proprietor: **IATRON LABORATORIES, INC.**
**11-4 Higashi-Kanda 1-chome**
**Chiyoda-ku**
**Tokyo 101 (JP)**

(72) Inventor: **SOE, Gilb**
**32-14, Ryukakujidai 4-chome, Sakae-cho**
**Inba-gun**
**Chiba 270-15 (JP)**
Inventor: **KOHNO, Isao Sukai-haitsu Futawa 201**
**57-11, Futawa-Higashi 5-chome**
**Funabashi-shi**
**Chiba 274 (JP)**
Inventor: **TANAKA, Michiyo**
**7-11, Miyanodai 2-chome**
**Sakura-shi**
**Chiba 285 (JP)**

(74) Representative: **Cohausz & Florack Patentanwälte**
**Postfach 33 02 29**
**D-40435 Düsseldorf (DE)**


EP 0 431 171 B1


Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**PATENT ABSTRACTS OF JAPAN**, vol. 12, no. 86 (P-677)(2933), 18 March 1988/

## Description

### TECHNICAL FIELD

The present invention relates to novel monoclonal antibodies specifically reactive to C-reactive protein, hybridoma cells secreting the monoclonal antibody, and immunoassay methods using the monoclonal antibody.

### BACKGROUND ART

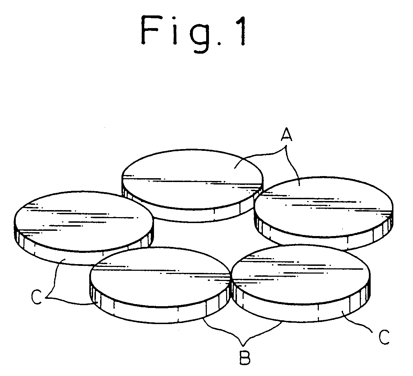

C-reactive protein (abbreviated as CRP) is one kind of acute phase proteins, and although its blood level rapidly increases on inflammatory diseases or the like accompanying the disorganization, it is present in healthy human blood only in a trace amount (at most 1 μg/ml). Therefore, it is widely used for the diagnosis of purulent diseases, rheumatosis and the like. As shown in Fig. 1, the CRP is a pentamer comprising five disk-like subunits (molecular weight about 20,000), and two monoclonal antibodies to the CRP have been constructed (The Journal of Immunology, Vol. 131, 2411-2 415). These are monoclonal antibodies which specifically react with the circular upper face of a disk-like subunit (A-face in Fig. 1; sometimes simply designated "A-face"), but does not react with the circular lower face of the disk-like subunit (B-face in Fig. 1; sometimes simply designated "B-face"); and a monoclonal antibody which reacts with the circular lower face (B-face) of a disk-like subunit, but does not react with the circular upper face (A-face) of the disk-like subunit. Nevertheless, a binding of the former monoclonal antibody or the latter monoclonal antibody to an insoluble carrier, followed by mixing with a CRP-containing sample, does not induce agglutination. Therefore, when the CRP is to be quantitatively measured using the above-mentioned known monoclonal antibodies, it is necessary to use a sandwich assay or the like utilizing both monoclonal antibodies.

Japanese Unexamined Patent Publication (Kokai) No. 62-218866 describes a reagent including an insoluble latex carrier sensitized with anti-human C-reactive protein antibody and a water-soluble compound selected from the group consisting of a trialkylamine, its salt and its quaternary ammonium salt. With incorporation of the trialkylamine compounds, it is possible to eliminate an error in determination of CRP when only a low CRP concentration is present in a sample. Although this document suggests that the anti-CRP antibody can be a polyvalent or monoclonal antibody, neither the preparation of a polyvalent or monoclonal antibody is indicated in that disclosure.

### DISCLOSURE OF THE INVENTION

The present inventors, as a result of investigations aimed at inducing an agglutination of the CRP using only one monoclonal antibody, found novel monoclonal antibodies which react with a portion of a disk-like subunit of the CRP different from the portions with which the known monoclonal antibodies react.

Accordingly, the present invention provides monoclonal antibodies which react with the side face of a disk-like subunit of the C-reactive protein (CRP) (C-face in Fig. 1; sometimes simply designated "C-face").

The present invention also provides monoclonal antibodies capable of inducing agglutination with C-reactive protein (CRP) on the basis of an antigen-antibody reaction.

Moreover, the present invention relates to hybridoma cells characterized in that they are constructed by a cell fusion of mouse myeroma cell with spleen cell of a mouse immunized by C-reactive protein (CRP), and secret a monoclonal antibody reactive with the side face of a disk-like subunit of a C-reactive protein.

Still further, the present invention relates to an immunoassay method for C-reactive protein (CRP) in a serum, characterized by using a monoclonal antibody which specifically reacts with the side face of a disk-like subunit of a C-reactive protein (CRP).

### BRIEF EXPLANATION OF THE DRAWINGS

Figure 1 schematically explains a structure of C-reactive protein (CRP); and
Fig. 2 is a graph showing the relationship between the agglutination rate and the CRP concentration.

### BEST MODE OF CARRYING OUT THE INVENTION

Monoclonal antibodies according to the present invention, which are monoclonal antibodies to the CRP, can be produced by culturing a novel mouse-hybridoma cells in vitro (for example, in a culture medium) or in vivo (for example, peritoneally in mice).

Mouse-hybridoma cells used herein are generally prepared by fusing a mouse myeloma cell with a spleen cell of a mouse immunized with CRP, according to the Köhler and Milstein method (see, Nature, Vol. 256, p 495, 1975).

As a medium for culturing the above-mentioned hybridoma cells, any medium suitable for culturing hybridoma cells can be used, but preferably Dulbecco's modified Eeagle's medium (abbreviated thereinafter as DME) containing fetal bovine serum, L-glutamine, L-pyruvate and antibiotics (penicillin G and streptomycin) is used.

The culturing of the above-mentioned hybridoma cells is carried out, for example, in a 5% $CO_2$ concentration at 37°C for about 3 days in the case of an in vitro culture, or, for example, peritoneally in a mouse for about 14 days in the case of an in vivo culture.

For an isolation and purification of a monoclonal antibody from a cultured broth or the ascites of mouse thus-prepared, any process generally used for the isolation and purification of a protein can be used. Such processes include salting out with ammonium sulfate, ion exchange chromatography, molecular sieve column chromatography using a molecular sieve gel, affinity column chromatography using protein A-bounded polysaccharide, dialysis, and lyophilization and the like.

The anti-CRP monoclonal antibody thus obtained has an ability to specifically react with only CRP, without reacting with other plasma proteins. In addition, the anti-CRP monoclonal antibodies according to the present invention specifically react with only the side face (C-face) but do not react with the circular upper face (A-face) or the circular lower face (B-face), of a disk-like submit. Moreover, since the anti-CRP monoclonal antibodies, when immobilized on an insoluble carrier, can induce agglutination with CRP, they are useful as an immunoassay reagent for a quantitative determination of CRP. For example, a CRP quantitative determination by agglutination uses only one anti-CRP monoclonal antibody of the present invention, which is bonded to a conventional insoluble carrier (for example, a latex such as polystylene latex particles) by a known chemical bounding method (using as a cross-linking agent, carbodiimide, glutaraldehyde or the like) or physical adsorption to form a complex. A known amount of the anti-CRP monoclonal antibody-bonded insoluble carrier complex and a predetermined amount of an aqueous sample (for example, serum, plasma, or urine) containing an unknown amount of CRP are brought into contact in a reaction cell on a slide plate or in an appropriate reaction container, and from an amount of agglutination thus formed, the CRP concentration can be quantitatively determined. For example, an agglutination reaction is determined visually using a slide plate, or spectro-photometrically at a particular wave length using a reaction cell.

Moreover, the present anti-CRP monoclonal antibody can be used in combination with another monoclonal antibody (for example, an monoclonal antibody specifically reacting with the circular upper face or circular lower face of a disk-like subunit) to carry out a quantitative immunoassay for various CRP-containing aqueous samples.

## Examples

Next, the present invention will be further illustrated by, but is no means limited to, the following examples.

### Example 1

(a) Preparation of immunized spleen cells

A CRP immunogen solution (PeL-Freez, US; A280 nm = 0.1) was mixed with an equal amount of Freund's complete adjuvant to form an emulsion, and 200 µl of this mixture was intraperitoneally administered to a mouse for immunization (the first immunization). After 30 days from the first immunization, 200 µl of the same mixture was intraperitoneally administered to the mouse (the second immunization). After 21 days from the second immunization, a CRP immunogen solution (A280 mm = 0.1) was diluted with an equal amount of physiological saline, and 200 µl of the diluted solution was intraveneously administered to said mouse (the last immunization). After 3 days from the last immunization, the spleen was aseptically removed from the mouse, to be used in the subsequent cell fusion step.

(b) Cell fusion

The spleen aseptically removed as described above was put into a petri dish containing 5 ml of DME medium supplemented with 15% fetal bovine serum. Next, the spleen was perfused with about 15 ml of DME supplemented with a 15% fetal bovine serum to wash out spleen cells, and the spleen cell suspension was passed through a nylon mesh. The spleen cell suspension was then collected in a 50 ml centrifugation tube, and centrifuged at 500 x g for 10 minutes. The cell pellet thus obtained was suspended in 4 ml of a hemolyzing solution (155 mM $NH_4Cl$, 10 mM $KHCO_3$ , 1mM $Na_2EDTA$, pH 7.0). Erythrocytes in the suspension were lyzed by allowing to put at 0°C for 5 minutes. After adding 10 ml of DME medium supplemented with 15% fetal bovine serum, the mixture was centrifuged, a resulting cell pellet was washed with DME medium by centrifugation, and viable spleen cells were counted.

On the other hand, 1 x $10^8$ of the above-prepared spleen cells were added to 2 x $10^7$ of previously cultured mouse myeloma SP 2/0-Ag 14 cells (Rikagaku Kenkyu Sho, Gene Bank), and were thoroughly mixed in a DME medium and centrifuged (500 x g, for 10 minutes). The supernatant was aspirated, the pellet was well slackened, 0.5 ml of 40% polyethylene glycol 4000 solution (kept at 38°C) was dropwise added thereon, and the centrifugation tube was manually and gently rotated for a minute, to mix the ethylene glycol solution and the cell pellet. Next, 1 ml per 30 seconds of DME medium kept at 38°C was added thereon, and the tube was manually and gently rotated. After repeating this procedure 10 times, 20 ml of a DME medium containing 15% fetal bovine serum was put thereon, and the mixture was centrifuged at 500 x g for 10 minutes. After eliminating the supernatant, the cell pellet was washed two times with a HAT medium (prepared by adding 4 x $10^{-7}$ M aminopterin, 1.6 x $10^{-5}$ M thymidine and 1 x $10^{-4}$ M hypoxanthine to DME medium) supplemented with 15% fetal bovine serum by centrifugation, and suspended in 40 ml of the above-mentioned HAT medium. Then, 200 µl of this cell suspension was distributed to each well of a 96-well cell culture plate, and culturing was started at 37°C in a carbon dioxide incubator containing 5% carbon dioxide. During the culturing, at an interval of 2 to 3 days, about 100 µl of the medium was eliminated from each well and about 100 µl of fresh HAT medium added thereon, to select hybridoma growing in the HAT medium. Starting from the eighth day, the medium was exchanged with HT medium (prepared by adding 1.6 x $10^{-5}$ M thymidine and 1 x $10^{-4}$ M hypoxanthine to DME medium) supplemented with 15% fetal bovine serum while monitoring the growth of hybridoma, and on the tenth day the hybridoma were screened for CRP productivity by an ELISA method as described below.

(c) Establishment of hybridoma

The presence or absence of an antibody in a hybridoma culture supernatant was determined by the ELISA method. First, 50 µl of a diluted CRP immunogen solution (A280 nm = 0.05, diluted with physiological saline) was distributed to each well of a 96-well ELISA plate (Immulon II, Nippon Dynatech K.K.), and the plate was allowed to stand at 25°C for 2 hours. Next, after washing three times with a 0.05% Tween$^R$ (trademark) 20-physiological saline, 50 µl of a culture supernatant was added to each well and a reaction was allowed at 25°C for one hour.

Next, 50 µl of peroxidase-linked mouse antibody (DAKO, Denmark) diluted 200 fold with a Tween$^R$ 20-physiological saline was added to each well. After completing the reaction, each well was washed three times with a 0.05% Tween$^R$ 20-physiological saline, and to each well was added 250 µl of a solution containing 0.5 mM aminoantipyrine, 10 mM phenol and 0.005% hydrogen peroxide, and after a reaction at 25°C for 30 minutes, the absorbance at 490 nm was measured for each well. As a result, 4 among 192 wells indicated the production of an antibody. Hybridoma cells in the 4 wells were transferred to 24-well plates, and cultured in an HT medium containing a 15% fetal bovine serum for 4 to 5 days, and after a further confirmation of the production of the anti-CRP antibody by the ELISA method, were cloned by a limiting dilution method. In the limiting dilution method, 100 µl of a cell suspension diluted with HT medium to 5 hybridoma cells/ml was distributed to each well of a 96-well plate, in which well 2 x $10^4$ peritoneal cells of normal BALB/C mouse had been distributed. Ten days later, hybridoma clones were screened for the production of an anti-CRP specific antibody, by the ELISA method, and as a result, 20 to 40 antibody-producing clones were obtained from each hybridoma. Among these clones, those clones which grew well, had a high ability to secret an antibody and were stable, were selected and re-cloned as described above to establish an anti-CRP specific antibody-producing hybridoma CRP-1, CRP-2, CRP-3 and CRP-4. These hybridoma were deposited with the Fermentation Research Institute Agency of Industrial Science and Technology on April 13, 1989, and have the following accession numbers.

| Hybridoma | Accession number |
|---|---|
| CRP-1 | FERM BP-2873 |
| CRP-2 | FERM BP-2874 |
| CRP-3 | FERM BP-2875 |
| CRP-4 | FERM BP-2876 |

Example 2 Production of monoclonal antibody

(a) In vitro process

The hybridoma CRP-1, CRP-2, CRP-3 and CRP-4 were separately cultured in DME medium containing a 15% fetal bovine serum at 37°C in a 5% carbon dioxide atmosphere for 72 to 96 hours. The culture was centrifuged (at 1000 x g, for 10 minutes), and to the supernatant was gradually added a solid ammonium sulfate, to make a final concentration of the ammonium sulfate 50%. The mixture was stirred under ice-cooling for 30 minutes, allowed to stand for 60 minutes, centrifuged (at 10,000 x g, for 10 minutes), a resulting precipitate was dissolved in a small amount of a 10 mM phosphate buffer (pH 8.0), and the solution was dialyzed against a 1000 times volume of 10 mM phosphate buffer. The dialyzate was filled in a DEAE-cellulose column equilibrated with 10 mM phosphate buffer, and a monoclonal antibody was eluted with a concentration gradient of between a 10 mM phosphate buffer (pH 8.0) and a 10 mM phosphate buffer (pH 8.0) containing 0.2 M NaCl. The eluted monoclonal antibody was concentrated by ultrafiltration, and dialyzed against a 0.1 M phosphate buffer (pH 8.0). To eliminate the bovine serum IgG, the dialyzate was passed through an anti-bovine serum goat IgG-Sepharose[R] 4B column. Next, the filtrate was filled in a protein A-Sepharose[R] 4B column equilibriated with a 0.1 M phosphate buffer (pH 8.0), and the column was subjected to elution with a buffer having a pH of 3.5 to obtain a solution of a purified anti-CRP specific antibody CRP-1 (this also applies to the CRP-2, CRP-3 and CRP-4).

(b) In vivo process

First, 0.5 ml of pristane[R] (2,6,10,14-tetramethylpentadecane) was intraperitoneally administered to BALB/C mice 10 to 12 weeks old, and then hybridoma CRP-1, CRP-2, CRP-3 or CRP-4 cells grown in vitro were inoculated to the mice in $2 \times 10^6$ cells per mouse.

For each hybridoma about 10 to 15 ml per mouse of the ascites was obtained. A concentration of antibody therein was 2 to 10 mg/ml. The monoclonal antibody in the ascites was purified by the same procedure as described above for the in vitro-purification (except that the anti-bovine serum goat IgG-Sepharose[R] 4B column step was omitted).

Example 3 Identification of immunoglobulin class and specificity of monoclonal antibody

The immunoglobulin class and specificity of the anti-CRP specific monoclonal antibodies CRP-1, CRP-2, CRP-3 and CRP-4 were determined by the Ouchterlony diffusion method and an enzyme immunoassay, respectively. The results are shown in Table 1.

Table 1

| Monoclonal Antibody | Immunoglobulin class |
|---|---|
| CRP-1 | $IgG_1$ |
| CRP-2 | $IgG_1$ |
| CRP-3 | $IgG_1$ |
| CRP-4 | $IgG_1$ |

Example 4 Binding of antibody to insoluble carrier (latex) and confirmation of reaction site

First, 2 ml of a latex solution (2%, Dow Chemical: diameter 0.482 µm) and 2 ml of a 2.0 mg/ml CRP-1 antibody aqueous solution were mixed and the mixture was stirred for about one hour. After centrifugation (at 20,000 x g for 10 minutes), precipitate was suspended in a 0.1% BSA solution, and the mixture stirred for about one hour. The mixture was again centrifuged (at 20,000 x g, for 10 minutes), the resulting precipitate was suspended in water, and the suspension was stirred for about two hours. In this manner, a suspension containing a CRP-1 antibody-latex complex was obtained. Similarly, suspensions containing a complex were obtained using the CRP-2 antibody, CRP-3 antibody and CRP-4 antibody, respectively.

On the other hand, a monoclonal antibody specifically reacting with the A-face of CRP but not reacting with the B-face (A-antibody), and a monoclonal antibody specifically reacting with the B-face of CRP but not reacting with the A-face (B-antibody) were prepared according to the Journal of Immunology Vol. 131, pp 2411-2415, and suspensions containing a complex were prepared as described above.

Next, 30 µl of the complex-containing suspension and 30 µl of a 25 µl/ml CRP solution were mixed on a slide glass, and the agglutination was visually observed. As a result, although an agglutination was not observed for the A-antibody and B-antibody, an agglutination was confirmed for CRP-1, CRP-2, CRP-3 and CRP-4 of the present invention. Accordingly, it is clear that the present CRP-1, 2, 3 and 4 antibodies are clearly different from a conventional antibody capable of specifically reacting with only the A-face and a antibody capable of specifically reacting with only the B-face.

Accordingly, a solution containing 20 µg/ml CRP masked at the A-face of the CRP with a corresponding antibody, and a solution containing 20 µg/ml CRP masked at the B-face of the CRP with a corresponding antibody were prepared, to 30 µl of the solution was added 30 µl of the above-mentioned antibody complex-containing suspension, and they were mixed on a slide glass. As a result, it was confirmed that the present CRP-1, 2, 3 and 4 antibodies induced the agglutination. This suggests that a specific reaction site of the present antibody is a site different from the A-face and B-face of CRP, and that since the present antibodies provide an agglutination by their reaction with CRP, the present antibodies specifically react with the side face of CRP.

Example 5 Quantitative assay by slide agglutination reaction

First, 30 µl of antibody latex complex suspension and 30 µl of an aqueous solutions containing different concentrations of CRP were mixed on a slide, and after shaking the slide for three minutes, an agglutination profile was visually observed. The results are shown in Table 2.

Table 2

| Antibody | CRP Concentration (µg/ml) | | | | | | | | | | | | |
|---|---|---|---|---|---|---|---|---|---|---|---|---|---|
| | 256 | 128 | 64 | 32 | 16 | 8 | 4 | 2 | 1 | 0.5 | 0.25 | 0.125 | 0.063 |
| CRP-1 | + | + | + | + | + | + | + | + | + | + | + | + | - |
| CRP-2 | + | + | + | + | + | + | - | - | - | - | - | - | - |
| CRP-3 | + | + | + | + | + | - | - | - | - | - | - | - | - |
| CRP-4 | + | + | + | + | + | + | + | + | + | + | - | - | - |

In Table 2, the symbols + and - denote the presence and absence of agglutination, respectively.

Example 6 Assay by spectrophotometry

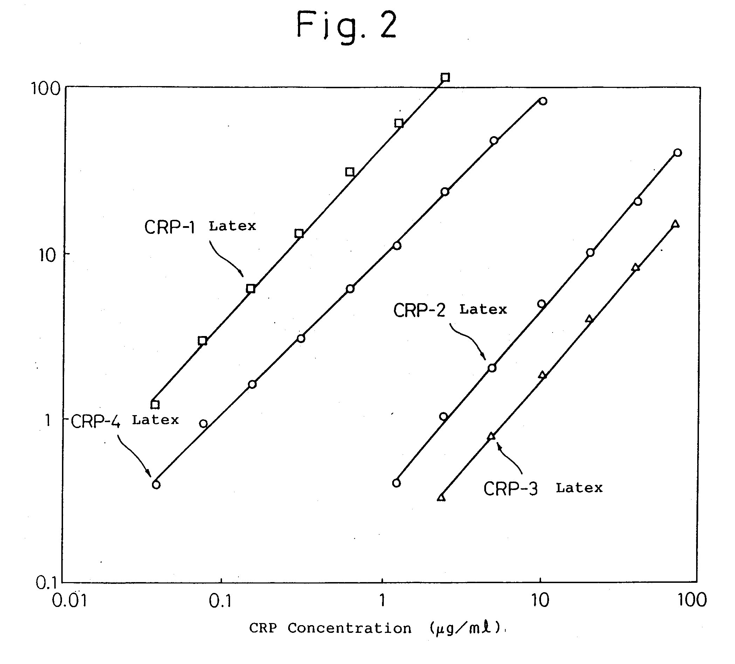

Latex (particle diameter 0.482 µm, Dow, Germany) coated with one of the monoclonal antibodies CRP-1, CRP-2, CRP-3 and CRP-4 prepared in Example 4 was used to test the human CRP concentration dependency of the agglutination rate, on a automatic analyzer (note: general name LPIAL-1; Mitsubishi Chemicals). As the human CRP, a product commercially available from PeL-Freez (US) was used, and 12 aqueous solutions containing human CRP at a concentration of 0.04 μg/ml to 80 μg/ml were prepared. The results are shown in Fig. 2. As seen from Fig. 2, each of the four later shows a CRP concentration-dependent agglutination rate (V value: change in transparency per time).

INDUSTRIAL APPLICABILITY

The present monoclonal antibodies are useful for measuring an amount of CRP in various samples.

Reference to microorganisms deposited under Rule 13-2, and depository authority: Fermentation Research Institute Agency of Industrial Science and Technology, the Ministry of International Trade and Industry

Address: 1-3 Higashi 1-chome, Tsukuba-shi, Ibaraki-ken, Japan

Deposition number and deposition date

1. FERM BP-2873 April 13, 1989
2. FERM BP-2874 April 13, 1989
3. FERM BP-2875 April 13, 1989
4. FERM BP-2876 April 13, 1989

**Claims**

1. A monoclonal antibody specifically reacting with the side face of a disk-like subunit of a C-reactive protein.

2. A monoclonal antibody capable of carrying an agglutination reaction due to an antigen-antibody reaction with the side face of a disk-like subunit of a C-reactive protein, when immobilized on an insoluble carrier.

3. A monoclonal antibody selected from the group consisting of monoclonal antibody CRP-1 obtainable from hybridoma cell line CRP-1 (FERM BP-2873), monoclonal antibody CRP-2 obtainable from hybridoma cell line CRP-2 (FERM BP-2874), monoclonal antibody CRP-3 obtainable from hybridoma cell line CRP-3 (FERM BP-2875), and monoclonal antibody CRP-4 obtainable from hybridoma cell line CRP-4 (FERM BP-2876).

4. A hybridoma cell characterized in that the cell is constructed by a cell fusion of a mouse myeloma cell and a spleen cell of a mouse immunized with a C-reactive protein, and secretes a monoclonal antibody specifically reacting with the side face of a disk-like subunit of a C-reactive protein.

5. A hybridoma cell line according to claim 4, selected from the group consisting of hybridoma cell line CRP-1 (FERM BP-2873), hybridoma cell line CRP-2 (FERM BP-2874), hybridoma cell line CRP-3 (FERM BP-2875), and hybridoma cell line CRP-4 (FERM BP-2876).

6. An immunoassay method for a C-reactive protein in the plasma, characterized by using a monoclonal antibody specifically reacting with the side face of a disk-like subunit of a C-reactive protein.

7. An immunoassay method according to claim 6, wherein the monoclonal antibody is selected from the group consisting of monoclonal antibody CRP-1 obtainable from hybridoma cell line CRP-1 (FERM BP-2873), monoclonal antibody CRP-2 obtainable from hybridoma cell line CRP-2 (FERM BP-2874), monoclonal antibody CRP-3 obtainable from hybridoma cell line CRP-3 (FERM BP-2875), and monoclonal antibody CRP-4 obtainable from hybridoma cell line CRP-4 (FERM BP-2876).

**Patentansprüche**

1. Monoklonaler Antikörper, der spezifisch mit der Seitenfläche einer scheibenartigen Untereinheit des C-reaktiven Proteins reagiert.

2. Monoklonaler Antikörper, der zur Durchführung einer Agglutination aufgrund einer Antigen-Antikörper-Reaktion mit der Seitenfläche einer scheibenartigen Untereinheit eines C-reaktiven Proteins bei Immobilisierung auf einem unlöslichen Träger befähigt ist.

3. Monoklonaler Antikörper, ausgewählt aus der Gruppe, bestehend aus dem monoklonalen Antikörper CRP-1, der aus der Hybridomazellinie CRP-1 (FERM BP-2873) erhältlich ist, dem monoklonalen Antikörper CRP-2, der aus der Hybridomazellinie CRP-2 (FERM BP-2874) erhältlich ist, dem monoklonalen Antikörper CRP-3, der aus der Hybridomazellinie CRP-3 (FERM BP-2875) erhältlich ist, und dem monoklonalen Antikörper CRP-4, der aus der Hybridomazellinie CRP-4 (FERM BP-2876) erhältlich ist.

4. Hybridomazellinie, dadurch gekennzeichnet, daß sie gebildet ist durch Zellfusion einer Mausmyelomazelle und einer Milzzelle einer Maus, die mit dem C-reaktiven Protein immunisiert worden ist und einen monoklonalen Antikörper absondert, der spezifisch mit der Seitenfläche einer scheibenartigen Untereinheit eines C-reaktiven Proteins reagiert.

5. Hybridomazellinie nach Anspruch 4, ausgewählt aus der Gruppe, bestehend aus der Hybridomazellinie CRP-1 (FERM BP-2873), der Hybridomazellinie CRP-2 (FERM BP-2874), der Hybridomazellinie CRP-3 (FERM BP-2875) und der Hybridomazellinie CRP-4 (FERM BP-2876).

6. Immunologisches Nachweisverfahren für ein C-reaktives Protein im Plasma, gekennzeichnet durch Verwendung eines monoklonalen Antikörpers, der spezifisch mit der Seitenfläche einer scheibenartigen Untereinheit eines C-reaktiven Proteins reagiert.

7. Immunologisches Nachweisverfahren nach Anspruch 6, worin der monoklonale Antikörper ausgewählt ist aus der Gruppe, bestehend aus dem monoklonalen Antikörper CRP-1, der aus der Hybridomazellinie CRP-1 (FERM BP-2873) erhältlich ist, dem monoklonalen Antikörper CRP-2, der aus der Hybridomazellinie CRP-2 (FERM BP-2874) erhältlich ist, dem monoklonalen Antikörper CRP-3, der aus der Hybridomazellinie CRP-3 (FERM BP-2875) erhältlich ist, und dem monoklonalen Antikörper CRP-4, der aus der Hybridomazellinie CRP-4 (FERM BP-2876) erhältlich ist.

**Revendications**

1. Anticorps monoclonal réagissant spécifiquement avec la face latérale d'une sous-unité en forme de disque d'une protéine C-réactive.

2. Anticorps monoclonal capable d'effectuer une réaction d'agglutination due à une réaction antigène-anticorps avec la face latérale d'une sous-unité en forme de disque d'une protéine C-réactive, lorsqu'il est immobilisé sur un support insoluble.

3. Anticorps monoclonal choisi parmi l'anticorps monoclonal CRP-1 pouvant être obtenu à partir de la lignée cellulaire d'hybridome CRP-1 (FERM BP-2873), de l'anticorps monoclonal CRP-2 pouvant être obtenu à partir de la lignée cellulaire d'hybridome CRP-2 (FERM BP-2874), de l'anticorps monoclonal CRP-3 pouvant être obtenu à partir de la lignée cellulaire d'hybridome CRP-3 (FERM BP-2875), et de l'anticorps monoclonal CRP-4 pouvant être obtenu à partir de la lignée cellulaire d'hybridome CRP-4 (FERM BP-2876).

4. Cellule d'hybridome caractérisée en ce qu'elle est construite par une fusion cellulaire d'une cellule de myélome de souris et d'une cellule de rate d'une souris immunisée avec une protéine C'réactive, et ce qu'elle secrète un anticorps monoclonal réagissant spécifiguement avec la face latérale d'une sous-unité en forme de disque d'une protéine C-réactive.

5. Lignée cellulaire d'hybridome selon la revendication 4, choisie parmi la lignée cellulaire d'hybridome CRP-1 (FERM BP-2873), la lignée cellulaire d'hybridome CRP-2 (FERM BP-2874), la lignée cellulaire d'hybridome CRP-3 (FERM BP-2875), et la lignée cellulaire d'hybridome CRP-4 (FERM BP-2876).

6. Méthode de dosage immunologique pour une protéine C-réactive dans le plasma, caractérisé en ce qu'on utilise un anticorps monoclonal réagissant spécifiquement avec la face latérale d'une sous-unité en forme de disque d'une protéine C-réactive.

7. Méthode de dosage immunologique selon la revendication 6, dans laquelle l'anticorps monoclonal est choisi parmi l'anticorps monoclonal CRP-1 pouvant être obtenu à partir de la lignée cellulaire d'hybridome CRP-1 (FERM BP-2873), l'anticorps monoclonal CRP-2 pouvant être obtenu à partir de la lignée cellulaire d'hybridome CRP-2 (FERM BP-2874), l'anticorps monoclonal CRP-3 pouvant être obtenu à partir de la lignée cellulaire d'hybridome CRP-3 (FERM BP-2875), l'anticorps monoclonal CRP-4 pouvant être obtenu à partir de la lignée cellulaire d'hybridome CRP-4 (FERM BP-2876).

Fig. 1

A
C
B
C

Fig. 2

CRP-1 Latex
CRP-2 Latex
CRP-3 Latex
CRP-4 Latex
CRP Concentration (μg/mℓ)
0.01
0.1
1
10
100
0.1
1
10
100